# EUROPEAN PATENT APPLICATION

(11) **EP 4 811 379 A1**
(43) Date of publication of application: **23.09.2026**
(21) Application number: 26165576.5
(22) Date of filing: 17.03.2026
(51) Int. Cl.: G16H 20/40

(54) **DYNAMIC ADJUSTMENT OF RADIOTHERAPY GRAPHICAL USER INTERFACES FOR PATIENT PRIVACY**

(30) Priority: 21.03.2025 US 202519087336
(71) Applicant: Varian Medical Systems, Inc., Palo Alto, CA 94304 (US)
(72) Inventor: SPATOLA, Brian, Palo Alto, 94304 (US); VOJAN, Filip, Palo Alto, 94304 (US)
(74) Representative: Mathisen & Macara LLP

(57) **Abstract**

A method for dynamically adjusting a graphical user interface (GUI) to preserve the privacy of a patient's personally identifiable information (PII) before, during, and/or after the administration of a radiotherapy treatment. The method may include displaying, on a screen 326 coupled to a gantry 324 of a radiotherapy machine 341, the GUI with one or more icons 308 representing the patient's PII. A privacy condition that is indicative of a heightened privacy risk of unauthorized viewing of the patient's PII is determined to be satisfied. In response to determining that the privacy condition is satisfied, the method may include adjusting the one or more icons of the GUI to anonymize the patient's PII to avoid unauthorized viewing of the patient's PII.

## Description

### TECHNICAL FIELD

This application relates generally to graphical user interfaces and software for radiotherapy machines and treatment planning, patient setup or treatment.

### BACKGROUND

Radiation therapy, which utilizes ionizing radiation, is a localized treatment targeting specific tissues, such as cancerous tumors. Ideally, this therapy focuses on the planning target volume (PTV) or target organ, sparing surrounding healthy tissue from excessive radiation doses to minimize damage. To ensure the prescribed dose is accurately delivered to the PTV, the patient must be precisely positioned relative to the linear accelerator, typically using a movable treatment couch on a turntable assembly. Implementing radiation therapy is a complex process involving specific guidelines, protocols, and instructions followed by various medical professionals, including clinicians, technicians, device manufacturers, and treating physicians. Due to the hazards associated with radiation, it is crucial that all instructions are meticulously followed.

Conventional radiation therapy systems are often complex to operate, requiring extensive training for efficient workflow execution. Tasks such as patient positioning, system calibration, and machine adjustments involve numerous interactions with both the patient and the radiotherapy machine. The data needed for these setups is often voluminous and complex. However, these conventional methods can be inefficient, ineffective, or even dangerous. Further, conventional radiotherapy systems do not provide sufficient privacy protections for the patient's personally identifiable information before, during, and/or after radiotherapy treatment alignment. By way of example, personally identifiable information of a patient receiving radiotherapy is continually visible on setup screens regardless of privacy risks arising during setup and administration of the radiotherapy.

### SUMMARY

Given the complexities and shortcomings of conventional radiotherapy systems discussed herein, there is a need for a graphical user interface (GUI) that provides intuitive and workflow-oriented information for a specific patient's radiotherapy treatments while preserving the privacy of the specific patient's personally identifiable information. Radiotherapy machines used during administration of radiotherapy treatments may include screens mounted to the radiotherapy machine to aid in the administration of the radiotherapy treatment. These screens may be mounted to a gantry of the machine, which is able to rotate around a patient during set up and delivery of the radiotherapy. The screen displays a GUI having multiple icons that provide details related to the radiotherapy process, such as setup parameters/adjustments, real-time positioning information, and/or patient information. Patient information presented on the screen may include the patient's personally identifiable information (PII).

In order to protect the patient's privacy before, during, and/or after the administration of the radiotherapy, the icons having the patient's PII are dynamically adjusted to anonymize the patient's PII when a privacy risk (also referred to herein as a privacy condition) arises. By way of example, during the alignment of the radiotherapy machine prior to administration of the radiotherapy treatment, the patient's name and picture are displayed on the screen to aid medical personnel quickly and accurately verify that the correct patient is receiving the correct treatment. If a door to the treatment room is opened during this alignment-when the patient's PII is visible on the screen-a privacy risk arises because someone without the proper authorization to view the patient's PII (e.g., technicians, cleaning personnel maintenance personnel, etc.) may enter the room and view the patient's PII. In response to the privacy risk, the patient's name and picture are anonymized into generic placeholders (e.g., a generic name and patient icon, respectively), thus preventing any unauthorized viewing of the patient's PII.

According to a first aspect of the invention, there is provided a computer-readable medium as defined in claim 1. According to a second aspect of the invention, there is provided a system as defined in claim 2. According to a third aspect of the invention, there is provided a method as defined in claim 3. Optional features are specified in the dependent claims. The display screen may be located on a gantry of the radiotherapy machine.

In one aspect, a method of treatment planning, patient setup or treatment monitoring comprises: presenting, by at least one processor on a display screen coupled to a radiotherapy machine, a set of icons corresponding to one or more attributes of a radiotherapy treatment planning, patient setup or treatment implemented via the radiotherapy machine; determining, by the at least one processor, that a privacy condition is satisfied; and in response to determining that the privacy condition is satisfied, anonymizing, by the at least one processor, at least one icon within the set of icons

The determination that a privacy condition is satisfied may be based on or in response to automatic detection of a trigger event. The radiotherapy machine may be housed in a room. The trigger event may be a change to the room, such as a door opening, an occupancy change, an environmental change, and/or the like. The trigger event may be generated by a sensor (e.g., occupancy sensor, proximity sensor, door sensor, light sensor, CO sensor, smoke detector, and/or sound sensor, etc.) and/or electronic devices. The sensors and/or electronic devices may transmit control or sensor signals to an analytics server (or part of the general system) to indicate a change in status (e.g., a door opening, lights turning on, and/or smoke detection, movement, etc.) so that it can be determined whether a privacy condition is satisfied. The analytics server (or part of the general system) may compare the transmitted signals to predefined privacy conditions and correlated triggers. If the transmitted signals received by the analytics server (or part of the general system) correspond to a trigger of the privacy condition, the analytics server (or part of the general system) may determine that the privacy condition is satisfied.

The set of icons on the display screen of the radiotherapy machine corresponding to one or more attributes of a radiotherapy treatment implemented via the radiotherapy machine, may include information that enables identification of a patient. This information may be automatically displayed initially to allow an operator to confirm the identity of the patient. The icons within the set of icons that are anonymized when the privacy condition is satisfied may be one of the icons that provide the information that enables identification of a patient. Such an arrangement allows a patient to be identified but reduces the likelihood that personally identifiable information of a patient will be inadvertently seen by unauthorized personnel.

A privacy condition may be automatically determined or detected, so that personally identifiable information of a patient may be automatically anonymized without relying on operator intervention. The operator may thus concentrate on the medical procedure, which may improve efficiency of the procedure.

In an embodiment, the techniques described herein relate to a computer-readable, medium comprising instructions, that when executed by one or more processors, cause the one or more processors to: present, on a display screen located on a gantry of a radiotherapy machine, a set of icons corresponding to one or more attributes of a radiotherapy treatment implemented via the radiotherapy machine; determine that a privacy condition is satisfied; and in response to determining that the privacy condition is satisfied, anonymize at least one icon within the set of icons.

The at least one icon within the set of icons may include personally identifiable information of a patient receiving the radiotherapy treatment or for whom radiotherapy treatment is planned.

The privacy condition may be a status change of a door of a treatment room including the radiotherapy machine. The privacy condition may be a clinician interacting with a pendant of the radiotherapy machine. The privacy condition may include identifying a person in a treatment room including the radiotherapy machine. The privacy condition may be an indication that the radiotherapy treatment has ended.

Anonymizing may include redacting at least one attribute of the at least one icon. Anonymizing may include relocating the at least one icon. Anonymizing may include blurring at least one attribute of the at least one icon.

In another embodiment, the techniques described herein relate to a system including: one or more processors; and a computer-readable, non-transitory storage medium containing instructions that when executed by the one or more processors cause the one or more processors to perform a method including: presenting, on a display screen located on a gantry of a radiotherapy machine, a set of icons corresponding to one or more attributes of a radiotherapy treatment implemented via the radiotherapy machine; determining that a privacy condition is satisfied; and in response to determining that the privacy condition is satisfied, anonymizing at least one icon within the set of icons.

The at least one icon within the set of icons may include personally identifiable information of a patient receiving the radiotherapy treatment or for whom radiotherapy treatment is planned.

The privacy condition may be a status change of a door of a treatment room including the radiotherapy machine. The privacy condition may be a clinician interacting with a pendant of the radiotherapy machine. The privacy condition may be identifying a person in a treatment room including the radiotherapy machine. The privacy condition may be an indication that the radiotherapy treatment has ended.

Anonymizing includes redacting at least one attribute of the at least one icon. Anonymizing includes relocating the at least one icon. Anonymizing includes blurring at least one attribute of the at least one icon.

In yet another embodiment, the techniques described herein relate to a method including: presenting, by at least one processor on a display screen located on a gantry of a radiotherapy machine, a set of icons corresponding to one or more attributes of a radiotherapy treatment implemented via the radiotherapy machine; determining, by the at least one processor, that a privacy condition is satisfied; and in response to determining that the privacy condition is satisfied, anonymizing, by the at least one processor, at least one icon within the set of icons.

The privacy condition may be a status change of a door of a treatment room including the radiotherapy machine.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2A** illustrates a radiotherapy machine in a default, upright orientation, according to an embodiment.
**FIG. 2B** illustrates a radiotherapy machine in a rotated orientation, according to an embodiment.
**FIG. 3A** illustrates a radiotherapy machine in a default, upright orientation and displaying a set of icons including a patient's personally identifiable information, according to an embodiment.
**FIG. 3B** illustrates a radiotherapy machine in a rotated orientation and displaying a set of icons anonymizing the patient's personally identifiable information, according to an embodiment.
**FIG. 4** illustrates a control pendant of the workflow-oriented radiotherapy system of **FIG. 1****,** according to an embodiment.
**FIG. 5** is a flow diagram of a method for dynamically adjusting a radiotherapy system's GUI to preserve a patient's privacy before, during, and/or after administration of a radiotherapy, according to an embodiment.
**FIGS. 6A-6C** illustrate a graphical user interface displayed on a radiotherapy machine, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein, as illustrated here, which would occur to a person skilled in the relevant art and having possession of this disclosure, are to be considered within the scope of the embodiments, methods, and/or systems described herein.

### System Architecture

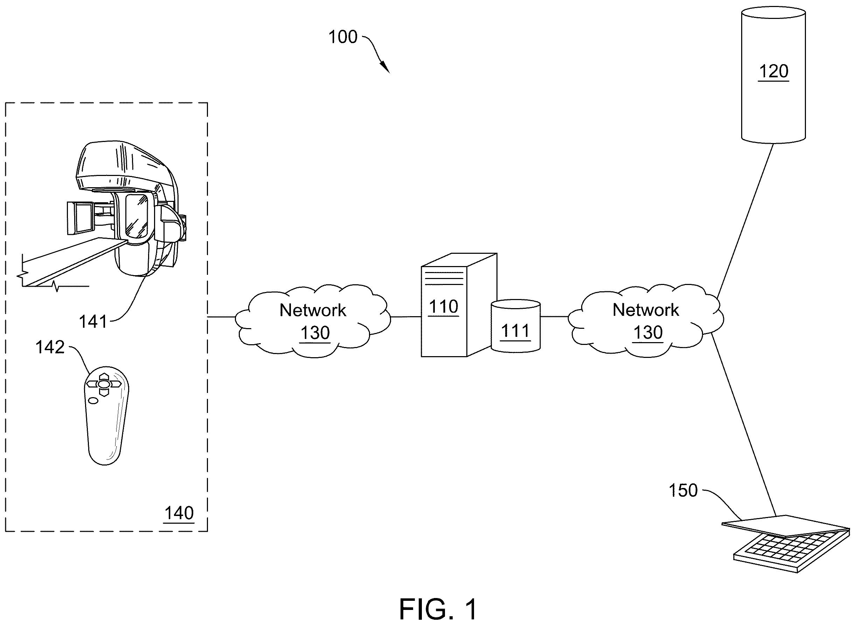

The methods described herein can be implemented using various computing devices/features described in **FIG. 1****.** Therefore, **FIG. 1** describes a non-limiting example of a computer environment where a server can perform the processes/methods described herein, such as retrieving, processing, and presenting radiotherapy treatment data, and presenting data for a graphical user interface (GUI) having workflow-oriented pages (or instances) on various displays screens.

**FIG. 1** illustrates various components of a system **100** for presenting various pages related to operation of a radiotherapy treatment, in accordance with an embodiment. The system **100** may include an analytics server **110,** a medical records database **120,** a radiotherapy system **140,** and a system administrator computer **150** or workstation. These features may communicate with each other over a network **130.** For example, the system **100** may present, via the analytics server **110,** one or more pages/GUIs on the radiotherapy machine **141.** In another example, the system **100** may present, via the analytics server **110,** a page presenting a live feed of a patient on one or more display devices, such as the display device on a gantry of the radiotherapy machine **141.**

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network **130** may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a computer-readable, non-transitory storage medium. The processing unit includes a processor with a computer-readable medium, such as a random-access memory coupled to the processor. In some embodiments, the analytics server **110** executes algorithms or computer executable program instructions, which may be executed by a single processor or multiple processors in a distributed configuration. The instructions allow the processor to implement the functionality described herein. The analytics server **110** may be configured to interact with one or more software modules of a same or a different type operating within the system **100.**

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, the **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****.** In alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111,** to store and/or retrieve various data described herein. For instance, the analytics server **110** may store different data corresponding to the different pages within the local database **111.** The page may be displayed on a display screen associated with the radiotherapy system **140.**

For instance, the analytics server **110** may display a page having a live feed of the patient before the treatment has begun or may display various pages describing how to position the patient on the couch and/or how to adjust the radiotherapy machine **141.** Even though some embodiments describe the analytics server **110** displaying various pages on a display screen attached to the radiotherapy machine **141** (e.g., located on the gantry of the radiotherapy machine **141),** it is expressly understood that the analytics server **110** may display different pages described herein on a display screen located anywhere within the treatment room, such as located on the wall of the treatment room or on any electronic device within the treatment room. In some configurations, the analytics server **110** may display the pages on the system administrator computer **150** or workstation.

The local database **111** may also store data corresponding to the radiotherapy machine **141** (e.g., default position of the radiotherapy machine **141,** current position of the radiotherapy machine **141,** such as the orientation of the bed/couch and gantry).

If the analytics server **110** receives a request from the radiotherapy system **140** to provide the current position/orientation of the radiotherapy machine **141,** the analytics server **110** may query the local database **111** and may be retrieve the corresponding data. Additionally, or alternatively, the analytics server **110** may also communicate with various sensors associated with the radiotherapy system **140** to retrieve machine data and parameters. The analytics server **110** may then use the methods/systems described herein to instruct the radiotherapy machine **141** to adjust the positioning of the radiotherapy machine **141** to the default position of the radiotherapy machine **141,** or another position of the radiotherapy machine, or to display one or more pages instructing a technician to adjust the radiotherapy machine **141** and/or the patient.

The local database **111** may also store data associated with different users of the radiotherapy system **140.** In a non-limiting example, the user data may include the user's login (e.g., sign-in, credentials) information and authorization levels. For example, the analytics server **110** may store, using the local database **111** may store, the user's name and password. The analytics server **110** may allow the user to log in to the radiotherapy machine **141,** and/or the system administrator computer **150.** If the user logs-in into the system, the analytics server **110** may adjust what the user is able to view, adjust, and control based on the user's authorization level. The analytics server **110** may conceal the patient's medical information that is not relevant to the radiation treatment from the medical technician. In another example, the analytics server **110** may conceal all of the patient's medical information if a machine technician signs-in.

The analytics server **110** may also utilize one or more other databases, such as the medical records database **120,** to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database **120** and may retrieve the corresponding dataset. The analytics server **110** may then use the methods/systems described herein to dynamically display the patient data in accordance with the patient identifier.

The medical records database **120** may also include a radiotherapy treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140,** the medical records database **120,** and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented, such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server 110 identifies the radiotherapy treatment file associated with the selected patient and customizes the graphical components described herein for the selected patient.

The analytics server **110** may also retrieve and instruct the medical records database **120** to store patient data associated with the patient from the medical records database **120,** the radiotherapy system **140,** and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data. For instance, the analytics server **110** may display the patient's profile image when the patient is selected, providing an opportunity to verify the correct patient was selected.

The analytics server **110** may receive treatment data associated with a patient from the medical records database **120,** the treatment data may further include at least a patient identifier. The analytics server may receive a radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy), or other non-treatment procedures, such as labs and appointments with other medical professionals. The radiotherapy treatment file may include data specific to one or more patient's radiotherapy treatment. The radiotherapy treatment file may include a patient identifier, patient's electronic health data records, medical images (e.g., CT scans, 4D CT scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index), and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141,** or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points), the analytics server **110** may execute various image recognition protocols and identify the tumor data.

Another example of a patient attribute may include specific tumor locations. More specifically, this data may indicate the primary tumor location with respect to the patient's centerline. This data may be inputted by the treating oncologist or may be analyzed using various image recognition or segmentation methods executed on the patient's medical images.

Another example of a patient attribute may include whether the patient uses prosthesis (e.g., hip or femoral head prosthesis). This attribute may result in a change of the patient's treatment (e.g., patients with these conditions might require a special treatment).

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system **100.** As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

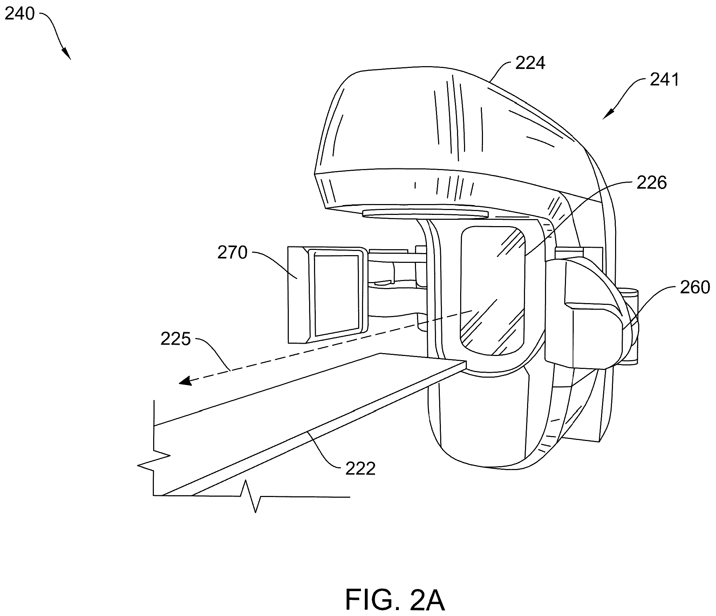

The radiotherapy machine **141** may also include a couch (shown as couch **222** in **FIG. 2A****)** to align the patient in a designated position before the treatment begins. The designated position may be identified, calculated, and/or retrieved by the analytics server **110.** For example, the analytics server **110** may retrieve patient data from the medical records database **120,** analyze the data, and utilize the analyzed data to position the patient on the couch. In some embodiments, the doctor identifies how to align the patient on the couch to optimize the treatment therapy. The radiotherapy machine **141** directs radiation at specified locations of the patient resting on the couch during treatment. The specified locations may be selected by the technician operating the radiotherapy machine **141,** the pendant **142,** and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposed to the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a set of cameras (e.g., camera on an end of the couch, gantry camera, ceiling camera, or other cameras placed within the radiotherapy room). The analytics server **110** may retrieve a live feed of the couch (e.g. with or without the patient) from the set of cameras and provide it to a display screen disposed on the radiotherapy machine **141.** The set of cameras may also directly communicate with the radiotherapy system **140.**

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of the set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The radiotherapy machine **141** may have a default home position. Before the patient receives treatment, the radiotherapy machine **141** may be reset to a position that is ergonomically desirable for the patient. The default home position may also be optimized to receive a new patient or allow the patient to more easily get off the couch.

As discussed in greater detail herein, the radiotherapy machine **141** may also be controlled by the pendant **142.** The pendant **142** may be connected to the radiotherapy machine **141** through wired or wireless communications according to, for example, Bluetooth specification sets, or another standard or proprietary wireless communication protocol. In various embodiments, the pendant **142** may be connected to the radiotherapy machine **141** through a wired connection or be integrated into the radiotherapy machine **141.** The pendant **142** may also be in communication with the analytics server **110.** The pendant **142** may adjust the positioning of the radiotherapy machine **141** through a selection of buttons that axially actuates the radiotherapy machine **141** (e.g., couch) towards a desired direction. The technician may also use the pendant **142** to initialize the radiotherapy machine **141,** and/or to have the radiotherapy machine **141** return to its home position.

The pendant **142** may also allow the technician to input patient information, which may be then communicated to the radiotherapy machine **141,** the analytics server **110,** and/or the medical records database **120.** The pendant **142** may be used to control one or more of the steps of the setup and/or treatment of the patient.

The pendant **142** may also include a touch pad (e.g., tactile sensor). The touch pad may be used to control various pages generated by the analytics server **110.** For example, the user may use the touch pad of the pendant **142** to control a page, generated by the analytics server **110,** to proceed through the workflow steps of the radiotherapy treatment and/or setup. In some embodiments, the page may be controlled through a touch screen on the radiotherapy machine **141** or elsewhere in the treatment room.

As discussed in greater detail herein, the radiotherapy system **140** may further include accessories that assist in the treatment and/or setup of the patient. The analytics server **110** may communicate with the radiotherapy system **140** to select which accessories are required. The analytics server **110** may also communicate with the medical records database **120,** and/or the system administrator computer **150** to determine which accessories are required. For example, a patient may require a specific accessory for their treatment and/or setup. The analytics server **110** may retrieve that the patient requires a specific accessory by accessing the medical records database **120** and communicate that information to the radiotherapy system **140.** The accessories may in communication with the radiotherapy system **140.** For instance, as will be described below, the analytics server may use RFID tags to scan and identify the location and/or presence of various accessories. In some embodiments, the accessories are wired or otherwise integrated into the radiotherapy machine **141.**

The local database **111** associated with the analytics server **110,** the medical records database **120,** and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110,** and the like. The medical records database **120,** may have a logical construct of data files, which are stored in computer-readable, non-transitory storage medium, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can monitor gantry movement, patient information, and modify various thresholds/rules described herein. The system administrator computer **150** may be configurable to display certain analytics metrics when specific thresholds/rules have been exceeded. For example, the system administrator computer **150** may be alerted if a patient has moved a specified amount during treatment and/or setup. The analytics server **110** may allow the system administrator computer **150** to also control and/or override the settings of the radiotherapy machine **141,** and/or the pendant **142.** For instance, an administrator may revise the minimum distance threshold and/or customize any feature on the pages described herein (e.g., move features within the page, color, font, shape, size, or the order of display for one or more pages).

The analytics server **110** may configure the system administrator computer **150** to review any and/or all commands made through the radiotherapy system **140** at specified process steps. The system administrator computer **150** may then allow or reject the commands made through the radiotherapy system **140.** For example, before the technician starts the patient treatment using the radiotherapy machine **141,** the analytics server **110** may first prompt the system administrator computer **150** to review the radiotherapy machine **141** parameters for approval. Once approved, the technician may then control the radiotherapy machine **141.**

The analytics server **110** may configure the system administrator computer **150** to review any and/or all reading and/or (over)writing of patient data to and/or from the medical records database **120.** For example, before the technician may (over)write patient information collected by the radiotherapy machine **141,** the analytics server **110** may first prompt the system administrator computer **150** to review the patient information before it is stored in the medical records database **120.**

As discussed in greater detail herein, the analytics server **110** may generate various pages that are designed to interact with the individual operating the radiotherapy machine **141,** and/or the system administrator computer **150.** The analytics server **110** may then generate various interactive pages and may instruct the network **130** to incorporate the generated pages displayed on the radiotherapy machine **141.**

### Dynamic Adjustment of Radiotherapy Display Screen to Anonymize Patient Information

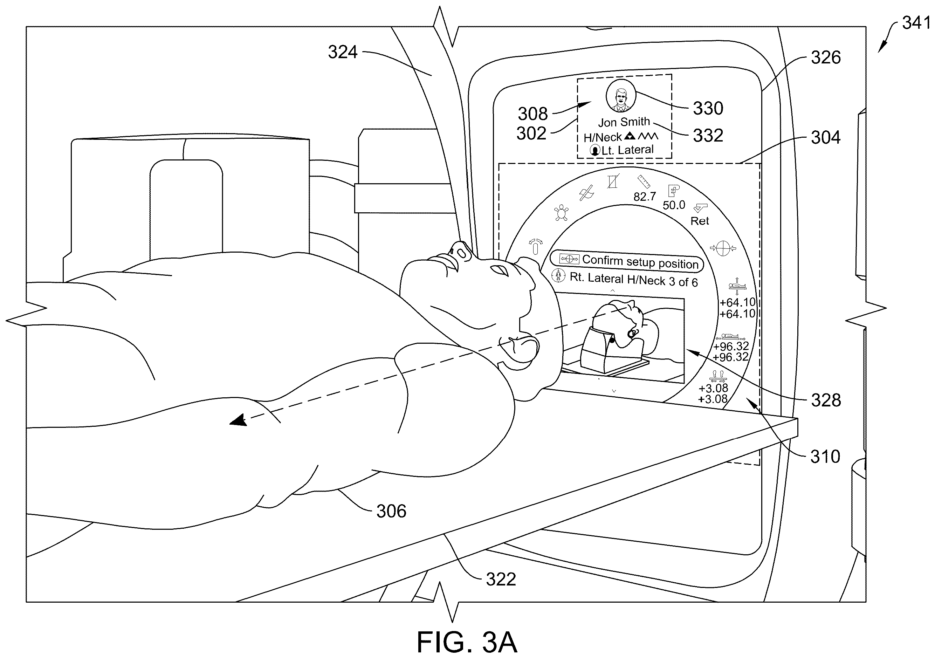

In at least one embodiment of the systems and methods described herein, the analytics server **110,** as described in **FIG. 1****,** may present for display one or more icons on a display screen coupled to the radiotherapy machine **141.** The one or more icons may be displayed as a GUI, as illustrated in **FIGS. 3A-3B****.** The one or more icons may present information related to the radiotherapy treatment and/or setup such as alignment information, therapy equipment needed, patient information, etc. Patient information presented on the screen may at times include the patient's personally identifiable information (PII).

In order to protect the patient's privacy before, during, and/or after the administration of the radiotherapy, icons that include the patient's PII are dynamically adjusted to anonymize the patient's PII when a privacy risk arises. For example, during the alignment of the radiotherapy machine prior to administration of the radiotherapy treatment, the patient's name and picture are displayed on the screen to aid medical personnel in quickly and accurately verifying that the correct patient is receiving (is about to receive) the correct treatment. While medical personnel administrating the therapy may be authorized to view the patient's PII displayed on the screen, a privacy risk may arise if, for example, a door to the treatment room is opened while the patient's PII is visible on the screen, because persons without the proper authorization to view the patient's PII (e.g., technicians, cleaning personnel, maintenance personnel, etc.) may enter the room. In response to the privacy risk, the patient's name and picture are anonymized into generic placeholders (e.g., a generic name and patient icon, respectively), thus preventing the unauthorized viewing of the patient's PII.

**FIG. 2A** illustrates a radiotherapy system **240** that may be configured to execute the privacy-protecting systems and methods described herein. The radiotherapy system **240** may be configured to anonymize (e.g., obfuscate, hide, remove, change, adjust, relocate, blur, obscure, replace, move) a patient's PII that is displayed on a screen when a privacy condition is satisfied (e.g., a door is opened, a privacy button is pressed, an unauthorized person enters the treatment room, etc.). The radiotherapy system **240** may include a radiotherapy machine **241** that includes a gantry **224** that rotates in a direction **228** (which may be clockwise or counterclockwise) about a center axis **225** of the radiotherapy machine **241.** In some embodiments, the radiotherapy system **240** may be substantially similar to the radiotherapy system **140** of **FIG. 1****.** Likewise, the radiotherapy machine **241** may be substantially similar to the radiotherapy machine **141** of **FIG. 1****.**

The radiotherapy machine **241** may include a display screen **226** positioned on or within the gantry **224.** The display screen **226** may be configured to display for view to an authorized user (e.g., a technician, a doctor, a patient, a medical practitioner) relevant information related to the radiotherapy procedure before, during, and/or after the radiotherapy procedure. The relevant information related to the radiotherapy procedure may be presented in a GUI with one or more sets of icons. The icons within the one or more sets of icons may include PII of a patient receiving the radiotherapy (e.g., patient **306** of **FIG. 3A****).** One or more processors (e.g., the analytics server **110)** may present for display the one or more sets of icons on the display screen **226,** or otherwise provide instructions that cause the display of the one or more sets of icons on the display screen **226.** While the analytics server is described herein as presenting icons on the display screen generally, it should be understood that the systems and methods described herein extend to any graphical component, such as, but not limited to, an indicator, figure, avatar, badge, symbol, marker, shape, object, emblem, arrow, direction, text, or any combination thereof. Indeed, as used in describing the methods and systems herein, the term icon may refer to any indicator, figure, avatar, badge, symbol, marker, shape, object, emblem, arrow, direction, text, color, number, or any combination thereof.

The radiotherapy machine **241** may also include an imager **270** and an emitter **260** for administering the radiotherapy to a patient. The radiotherapy system **240** includes a couch **222,** upon which the patient may be placed before and/or during the administration of the radiotherapy (e.g. for imaging by imager 270 prior to radiotherapy treatment). In some embodiments, the couch **222** may be translated laterally relative to the radiotherapy machine **241** (e.g., along the center axis **225)** and/or rotated axially relative to the center axis **225** of the radiotherapy machine **241.** Likewise, the radiotherapy machine **241** may be rotated axially about the center axis **225,** and relative to the couch **222,** before, during, and/or after the administration of the radiotherapy treatment.

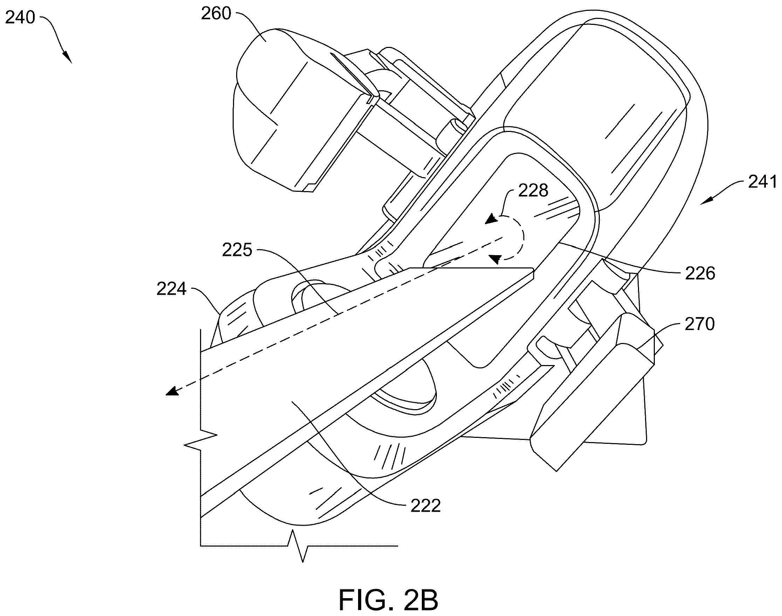

For example, as illustrated in **FIG. 2B****,** the radiotherapy machine **241** is shown in a rotated position about the center axis **225.** Relative to the default position of the radiotherapy machine **241** (as shown in **FIG. 2A****),** the radiotherapy machine **241** in **FIG. 2B** is shown at a position that is rotated about 135.5° counterclockwise. Because the display screen **226** is coupled to the gantry **224** of the radiotherapy machine **241,** the display screen **226** is also rotated from a default, upright position (as shown in **FIG. 2A****)** at about 135.5° counterclockwise. Though the gantry **224** and the display screen **226** are shown rotating counterclockwise, it is understood that the gantry **224** and the display screen **226** may additionally or alternatively rotate clockwise from the default position shown in **FIG. 2A****.**

During a radiotherapy treatment and/or setup procedure, as shown in **FIG. 3A****,** a patient **306** is placed upon the couch **322** (which may be substantially similar to the couch **222** of **FIG. 2A****),** which is positioned near the radiotherapy machine **341** (which may be substantially similar to the radiotherapy machine **241** of **FIG. 2A****).** Before, during, and/or after administration of the radiotherapy treatment, an analytics server (e.g., the analytics server **110** of **FIG. 1****)** may present for display a set of icons (e.g., graphical/textual icons) on the display screen **326** (which may be substantially similar to the display screen **226** of **FIG. 2A****).** In some embodiments, the icons displayed may be grouped into two separate subsets. The grouping may be performed using predefined criteria (e.g., in accordance with the content or location of the icon or icons). In some embodiments, one subset (e.g., one or more icons) may appear to rotate with the gantry **324** (which may be substantially similar to the gantry **224** of **FIG. 2A****)** and the display screen **326,** while another group of icons may appear to be stationary.

In a non-limiting example, a first subset of icons (e.g., the first subset of icons **308** depicted in **FIG. 3A****)** may be presented for display at a first position **302** of the display screen **326.** A second subset of icons **310** (e.g., the second subset of icons **310** of **FIG. 3A****)** of the set of icons may be alternatively, or additionally, presented for display in an area **304** on the display screen **326.**

In at least one embodiment, the first subset of icons **308** includes PII related to the patient **306** and/or the radiotherapy treatment and/or setup. For example, the first subset of icons **308** may include a visual graphic of the patient **306** (shown as photo **330),** such that a medical practitioner may visually confirm an identity of the patient **306** relative to the photo **330** and accurately verify that the radiotherapy treatment being administered (or about to be administered) is the correct radiotherapy treatment. Additionally, or alternatively, the first subset of icons **308** may include text **332** related to the patient **306.** As shown in **FIG. 3A****,** the text **332** represents a name of the patient **306.** While text **332** illustrates the name of the patient **306** in **FIG. 3A****,** it should be understood that the text **332** may include non-PII data and/or other types of PII of the patient **306,** such as a name, age, height, weight, type of treatment to be administered, emergency contact information, insurance information, a Social Security number, driver's license numbers, passport details, addresses, email addresses, phone numbers, credit card or bank account information, biometric data (such as fingerprints or facial recognition data), medical records, dates of birth, employment records, and/or educational records.

The first subset of icons **308** may also include non-PII icons/information. Non-PII icons/information may include, for example, a type and/or location of the therapy to be administered to the patient **306** (e.g., "LT Lateral"). Because the type and/or location of the therapy does not personally identify the patient **306,** it is considered non-PII.

The second subset of icons **310** may include information related to the setup of the radiotherapy machine **341,** the couch **322,** and/or the radiotherapy treatment more generally, which may or may not include any PII. The second subset of icons **310** may include a group of icons related to patient setup and accessories, rotational parameters of the radiotherapy machine **341,** couch parameters, and/or an interlocks/system status. The second subset of icons **310** may include visual and/or text graphics related to a current position of the radiotherapy machine **341** and/or the couch **322,** and a desired position of the radiotherapy machine **341** and/or the couch **322.** In some embodiments, the second subset of icons **310** includes visual and/or text/numerical indicators that indicate a positional change to be applied to one or more of the couch **322** and/or the radiotherapy machine **341,** such that the couch **222** is appropriately aligned relative to the radiotherapy machine **341** for administration of the radiotherapy treatment. In some embodiments, one or more icons within the second subset of icons **310** may be dynamically updated as the radiotherapy machine **341** and/or the couch **322** are positionally adjusted.

In at least one embodiment, the second subset of icons **310** includes PII of the patient **306.** For example, the second subset of icons **310** may include icon **328.** Icon **328** is illustrated in **FIG. 3A** as a setup image illustrating the proper placement of the patient **306** for administration of the radiotherapy. In some embodiments, the icon **328** is a real-time (or substantially real-time) image of the patient **306.** In some embodiments, the icon **328** is a static photo of the patient **306** in the proper alignment for the radiotherapy. While **FIG. 3A** depicts icon **328** as a setup image for aiding in placement of the patient **306,** it is understood that icon **328** may be any PII of the patient **306,** including text or graphical images.

The first subset of icons **308** and/or the second subset of icons **310** may have one or more icon parameters. For example, the icon parameters of first subset of icons **308** and/or the second subset of icons **310** may include a color, font type, font style, font size, font weight, font color, alignment, spacing, letter spacing, text case, orientation, opacity, text direction, background color, text padding/margin, shadow, text wrapping, text scaling, text rotation, border/outline, underline/bold/strikethrough, and/or animation. The icon parameters may be used by the analytics server in displaying the first subset of icons **308** and/or the second subset of icons **310** to define the look and feel of the first subset of icons **308** and/or the second subset of icons **310.**

While the first subset of icons **308** and/or the second subset of icons **310** are displayed on the display screen **326,** the analytics server may monitor (e.g., periodically) one or more privacy conditions (e.g., privacy risks). As used herein, a privacy condition relates to a situation in which there is a heightened risk of non-authorized viewing of PII of the patient **306.** The privacy condition may be caused by one or more triggers (e.g., events, signals, inputs, status changes, etc.).

Non-limiting examples of triggers of the privacy condition include a door opening, unauthorized user detection (e.g., identifying an unverified individual entering the room via RFID badges, facial recognition, or motion sensors); a pendant interaction (e.g., a clinician pressing a privacy button on a pendant such as the pendant **142** of **FIG. 1****);** room occupancy sensor detection; patient presence change (e.g., when the patient leaves the treatment table or room, or a new patient enters before proper setup); a system timeout (e.g., after a period of inactivity on the screen; scheduled privacy mode (e.g., based on predefined schedules, such as during non-operational hours or staff shift changes); screen sharing or mirroring (e.g., when the screen is detected by the analytics server to be shared or mirrored to external devices); unauthorized device detection (e.g., an unapproved electronic device, such as a camera or smartphone, is detected in the room); emergency stop or alarm activation (e.g., an emergency stop of the machine or activation of a safety alarm); geofencing or proximity detection (e.g., a location of authorized personnel leaving the immediate vicinity or an unauthorized personnel arriving at the immediate vicinity); workflow transition (e.g., transitions between patient setups, switching to general maintenance or cleaning workflows, at the end of a treatment); manual override by staff (e.g., a user interaction with a control panel or remote such as the pendant **142** of **FIG. 1****);** live monitoring activation (e.g., the room is being monitored remotely by other staff or systems); treatment status change (e.g., certain stages of the treatment process, such as at the end of the treatment preparation or when entering standby mode); compliance check failures (e.g., a compliance issue is detected, such as unsecured patient data or workflow errors); and/or environmental changes (e.g., sudden lighting changes-such as lights turning on/off-suggesting someone entered the room unexpectedly).

The analytics server may be communicably coupled to one or more communication devices (e.g., the pendant **142** and/or the system administrator computer **150** of **FIG. 1****),** sensors (e.g., occupancy sensor, proximity sensor, door sensors, light sensor, CO sensor, smoke detector, sound sensor, etc.) and/or electronic devices. The communication devices, sensors, and/or electronic devices may transmit control or sensor signals to the analytics server to indicate a change in status (e.g., a door opening, lights turning on, smoke detection, and/or movement, etc.). The analytics server compares the transmitted signals to predefined privacy conditions and correlated triggers. If the transmitted signals received by the analytics server correspond to a trigger of the privacy condition, the analytics server determines that the privacy condition is satisfied.

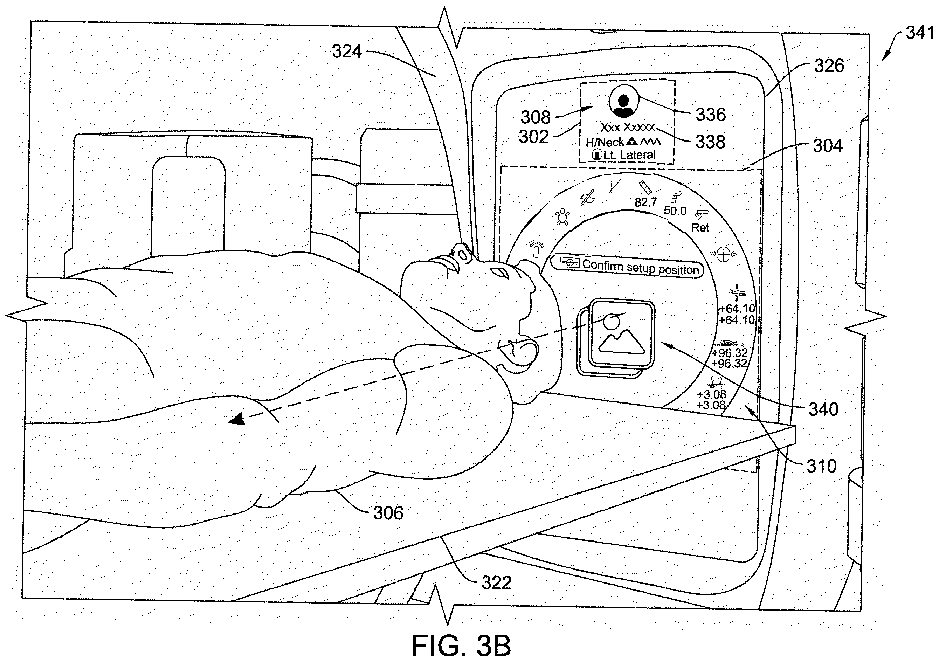

As shown in **FIG. 3B****,** in response to the analytics server determining that a privacy condition is satisfied due to a trigger occurring (as described above), the analytics server anonymizes at least one icon within the set of icons so as to protect the patient's PII from unauthorized viewing. The analytics server may anonymize the at least one icon with one or more methods. For example, the analytics server may transmit control signals to the display screen **326** adjust one or more icon attributes/parameters/content to redact, relocate, blur, obfuscate, mask, move, remove, change, encrypt, adjust, replace, or a combination thereof, the at least one icon having the patient's PII.

The analytics server may transmit controls to redact the at least one icon. In at least one implementation, the analytics server may replace the PII with placeholders. For example, the photo **330** of **FIG. 3A** is replaced with the generic avatar **336** of **FIG. 3B****.** The text **332** of **FIG. 3A** is replaced with the generic text **338** of **FIG. 3B****.** The icon **328** of **FIG. 3A** is replaced with the generic photo icon **340** of **FIG. 3B****.** In some embodiments, the analytics server transmits a control signal to blur the photo **330,** the text **332,** and/or the icon **328** such that they are illegible. This may include adjusting one or more of the icon parameters to cause the photo **330,** the text **332** and/or the icon **328** to blur. In some embodiments, the analytics server transmits a control signal to reduce an opacity of the icons such that they are no longer visible on the display screen **326.** The analytics server may transmit an instruction to adjust a color of the at least one icon (e.g., the font color) to match the background color such that the icon is not visible on the display screen **326.** In some embodiments, the analytics server transmits an instruction to mask the PII of the at least one icon with another graphic such that the PII of the at least one icon is not visible. In some embodiments, the analytics server relocates the at least one icon to an area not visible on the display screen **326.** By way of example, the at least one icon may be relocated to a portion of the display screen **326** hidden behind a display shield, a body panel of the gantry **324,** etc. In some embodiments, the analytics server transmits instructions to remove from display some or all of the icons containing the patient's PII.

In a non-limiting illustrative example, the treatment room in which the radiotherapy machine **341** is located may have a door monitored by a sensor (e.g., a proximity sensor that monitors a location of the door relative to a frame of the door). The sensor may be communicably coupled to the analytics server, which monitors the status of the door (e.g., open/closed) based on a determination made in response to communications transmitted by the sensor. For example, the sensor may transmit a signal to the analytics server that the door has moved away from the frame beyond a threshold distance (e.g., 1-12 inches, 25.4 - 305 mm). The analytics server receives this transmitted signal and determines that the door has changed from a closed status to an open status. The analytics server determines that a privacy condition (e.g., "open door" status) is satisfied based on the changing door status. If, prior to the change from closed status to open status, PII of the patient **306** was being displayed, the analytics server responds (based at least in part on the privacy condition being satisfied) by anonymizing the PII of the patient **306** by executing one or more of the anonymizing methods described herein. Alternative or additional status changes that may trigger/satisfy the privacy condition may include lights going from off to on; a monitor going from not screen mirroring to screen mirroring; the presence of an unauthorized person within a viewing area; change from non-emergency to emergency; active session changing to standby mode; a setup mode changing to therapy administration mode; privacy button being clicked, etc. Various privacy conditions associated with the triggers above may exist and be satisfied to cause the analytics server to anonymize the PII. Additional or alternative privacy conditions may include an unauthorized user present in the treatment room, screen mirroring, remote monitoring (e.g., by video/image transmission), standby mode, privacy mode, etc.

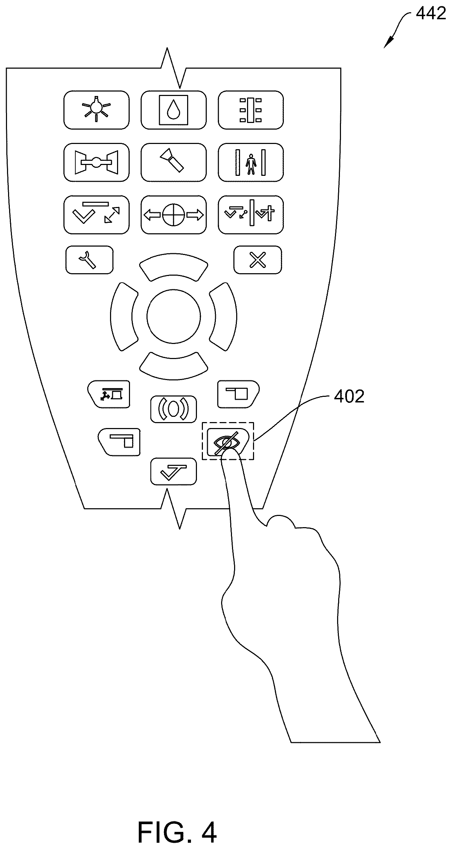

In a non-limiting embodiment, the radiotherapy system may include a pendant, such as a pendant **442** of **FIG. 4****.** The pendant **442** may be substantially similar to the pendant **142** of **FIG. 1****.** A radiotherapy machine (e.g., the **342** of **FIGS. 3A-3B****)** may also be controlled by the pendant **442.** The pendant **442** may be connected to the radiotherapy machine through wired or wireless communications according to, for example, Bluetooth specification sets, or another standard or proprietary wireless communication protocol. In various embodiments, the pendant **442** may be connected to the radiotherapy machine through a wired connection or be integrated into the radiotherapy machine. The pendant **442** may also be in communication with the analytics server. The pendant **442** may adjust the positioning of the radiotherapy machine **141** through a selection of buttons that axially actuate the radiotherapy machine (e.g., couch) towards a desired direction. The clinician (e.g., a technician) may also use the pendant **442** to initialize the radiotherapy machine, and/or to have the radiotherapy machine return to its home position.

The pendant **442** may also allow the clinician to input patient information, which may be then communicated to the radiotherapy machine, the analytics server, and/or the medical records database (e.g., the medical records database **120** of **FIG. 1****).** The pendant **442** may be used by the clinician to control one or more of the steps of the setup and treatment of the patient.

The pendant **442** may also include a touchpad (e.g., tactile sensor). The touchpad may be used by the technician to control various pages generated by the analytics server. For example, the technician may use the touchpad of the pendant **442** to control a page, generated by the analytics server, to proceed through the workflow steps of the radiotherapy treatment and/or setup. In some embodiments, the page may be controlled through a touchscreen on the radiotherapy machine or elsewhere in the treatment room.

The pendant **442** may be used to initiate a privacy mode of the radiotherapy system. The privacy mode may include anonymizing the patient's PII such that it no longer is personally identifying. For example, if the clinician feels that the patient's PII is at risk of unauthorized viewing, the clinician may interact with button **402** of **FIG. 4** to initiate the privacy mode. Upon the pendant **442** receiving an indication of an interaction with the button **402,** the pendant **442** transmits a data signal to the analytics server corresponding to the interaction. The analytics server queries the current mode (e.g., privacy mode or non-privacy mode) and toggles the mode to the non-current mode. For example, if the current mode is non-privacy mode (e.g., the patient's PII is displayed and visible), the analytics server toggles the mode to privacy mode (e.g., anonymizing the patient's PII) in response to receiving the data signal of the button **402** interaction from the pendant **442.**

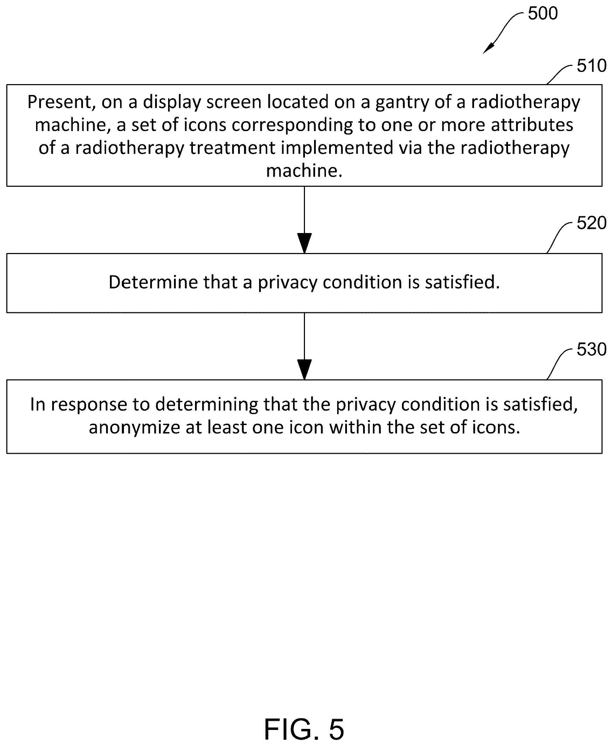

**FIG. 5** is a flowchart of an example method for protecting a patient's PII. The method **500** may be executed by one or more processors described herein, such as the analytics server **110,** the pendant **142,** the system administrator computer **150,** the radiotherapy system **140,** the radiotherapy machine **141,** etc. The one or more processors may execute instructions stored on a computer-readable, non-transitory storage medium, which may cause the one or more processors to execute steps of the method **500.** While the steps of the method **500** are shown in a specific order, it should be understood that the method **500** may comprise more, fewer, and/or different steps than those depicted in **FIG. 5****.** Likewise, the order of the steps of the method **500** is shown in **FIG. 5** for illustrative purposes. However, it is understood that the steps of method **500** may be performed in any number of configurations or orders without departing from the scope of the systems and methods described herein.

At step **510,** at least one processor presents on a display screen located on a gantry of a radiotherapy machine, a set of icons corresponding to one or more attributes of a radiotherapy treatment and/or setup implemented via the radiotherapy machine. The set of icons may include any number of subsets of icons, such as the first subset of icons **308** and/or the second subset of icons **310** of **FIG. 3A****.** In general, any of the steps discussed herein as performed by the analytics server may be performed by the system in general (i.e. no separate analytics server is required).

In some embodiments, the one or more processors may present, on a display screen located on the gantry of a radiotherapy machine, a set of icons corresponding to a set of tasks needed to implement/facilitate a patient's radiotherapy treatment. As discussed herein, e.g., **FIGS. 2A-B****,** the display screen may be integrated into the gantry and may serve as an intuitive and interactive interface for operators, providing task-specific visual elements that guide the execution of various stages of the treatment process. Each icon may represent a distinct instruction or task within the treatment workflow, such as patient alignment, equipment setup, dose calibration, or verification of treatment parameters.

In some embodiments, the one or more processors dynamically update the display to ensure the presented icons are relevant to the various stages of the radiotherapy treatment. In some embodiments, the one or more processors may generate a set of tasks for configuring a radiotherapy treatment for a patient, with each task associated with a specific stage of the treatment. This structured and workflow-oriented approach ensures that the radiotherapy process is methodical and tailored to the patient's specific needs. To initiate the process, the processors may retrieve a radiotherapy file (RT file) associated with the patient, which contains critical treatment data. The RT file serves as a comprehensive repository of information necessary for planning, executing, and monitoring the radiotherapy treatment. It may include patient identifiers such as name and demographic details, imaging data (e.g., CT scans, MRIs, x-rays), and detailed treatment plans, including beam trajectories, dose levels, arc information, and radiotherapy types. Additionally, it specifies target and non-target organ data to ensure precise radiation delivery while minimizing exposure to healthy tissues.

The RT file may also contain patient-specific attributes like physical characteristics (e.g., height, weight, BMI), medical history, and session-specific details such as prior couch positioning adjustments. It may include required accessory details, like immobilization devices or bolus materials, enabling consistency across treatment sessions. Leveraging this file, the processors dynamically generate workflow tasks, populate the GUI with task-relevant values, and provide real-time updates as tasks are completed. These tasks correspond to various stages of the radiotherapy workflow, such as patient alignment, machine calibration, accessory verification, dose administration, and post-treatment analysis. For instance, during the alignment stage, tasks may guide the operator in adjusting the couch and gantry to achieve proper alignment relative to the machine's isocenter. As tasks are completed, the processors or servers update the icons displayed on the GUI to reflect task status, ensuring seamless progression through the workflow. For example, processors may detect a completed task, such as couch movement, and update the corresponding icon, or operators may input task completion using a pendant, prompting the processors to adjust the GUI accordingly.

In some embodiments, the icons may be designed with visual attributes, such as color, shape, or animations, that reflect the status of each instruction, providing operators with immediate feedback on task completion or required actions. This dynamic and task-specific presentation enhances usability, reduces cognitive load, and ensures a streamlined and error-resistant workflow for delivering precise and effective radiotherapy treatment.

After the tasks to be performed by the operator have been identified, the one or more processors may display the set of icons. In some embodiments, the set of icons may be radially arranged. That is, the icons may be radially arranged around a center point on the display screen, allowing for uniform distribution and simultaneous visibility, regardless of the screen's orientation or the gantry's position. This radial configuration may enhance accessibility and usability, enabling operators to efficiently locate and interact with icons without diverting attention from the patient or machine. In some embodiments, the icons can also be grouped based on predefined attributes, such as workflow stage or type of information, with customization options available to adapt groupings to the preferences of different operators or clinics.

Furthermore, the icons are dynamically interactive, responding to changes in the treatment environment and user inputs. For instance, once the patient alignment is verified, the corresponding icon may change color or display a checkmark to indicate completion, while the next icon highlights instructions for dose calibration. These dynamic and customizable features provide operators with an intuitive and efficient interface that reduces cognitive load, improves workflow precision, and enhances the overall radiotherapy treatment process.

As depicted, the set of displayed icons includes one or more icons that may include the patient's personally identifiable information, such as the patient's image, name, diagnoses, and other information that can identify the patient. Different groupings of icons (or sometimes individual icons) may be arranged in accordance with a predefined order that may or may not correspond to the workflow of the patient's radiotherapy treatment. For instance, in one embodiment, different tasks to be performed may be associated with a defined order. As a result, their corresponding icons may be arranged in that order.

In other embodiments, such as the depicted embodiment, the icons may be arranged in accordance with a grouping of the tasks. For instance, icons may be arranged based on whether they are associated with a patient setup category, radiotherapy accessory category, radiotherapy machine attribute category, rotational parameter category, interlocks, or a system status category. As depicted, the set of icons may be divided into three groups where the groups indicate a cluster of tasks to be performed. The cluster of tasks may have a common attribute and may be clustered based on the common attribute. For instance, one grouping or cluster of icons may indicate adjustments to be made to the patient (e.g., patient alignment) and another grouping of the icons may be directed to machine adjustment.

A first grouping of the icons may include patient alignment indicators. A second grouping of icons may include machine/treatment accessories. Another grouping of the icons may include machine attribute icons that correspond to tasks related to adjusting the radiotherapy machine.

In some embodiments, system administrators or operators may have the capability to adjust any attribute of the icons displayed on the display screen, including visual attributes such as color, font style, size, line thickness, shading, and the like. This flexibility allows the icons to be modified to align with institutional branding, operator preferences, or accessibility requirements. For instance, line thickness or font size can be increased to enhance visibility for users with visual impairments. These adjustments ensure that the interface remains both functional and user-friendly, tailored to meet the specific needs of the clinical setting.

The position of the icons on the display screen can also be adjusted by system administrators or operators to optimize usability and align with operator workflows. Icons can be repositioned within the radial arrangement or moved to alternative layouts, ensuring that the most frequently accessed icons are conveniently located. This customization enables the interface to adapt to specific clinic requirements or individual user preferences, improving efficiency during radiotherapy procedures. For instance, a clinic focusing on particular types of treatments may prioritize the placement of certain icons for quick access, minimizing navigation time and enhancing operational precision.

In some embodiments, a system administrator or an operator can be authorized to add new ones to better reflect the workflow or accommodate updates to the radiotherapy process. This capability ensures that the interface remains relevant and streamlined, reducing visual clutter and focusing only on the tasks and information pertinent to the specific clinical scenario. For example, an icon can be added to the set of icons that indicates whether a door of the treatment room is open, whether a predicted collision is going to occur, whether the pendant is properly connected to the radiotherapy machine, and the like.

At step **520,** the at least one processor determines that a privacy condition is satisfied. As described above, the privacy condition may refer to a condition in which there is a heightened risk for unauthorized viewing of the patient's PII. The one or more processors may be communicably coupled to one or more electronic devices, communication devices, and/or sensors which transmit data/control signals to the one or more processors that are indicative of a status change of one or more attributes, such as a door position, an operating mode, a personnel location, etc. The data/control signals transmitted to the one or more processors may act as triggers for determining that the privacy condition is satisfied.

Once the one or more processors receive the data/control signal, the one or more processors may toggle a bi-modal status (e.g., door open/door closed) or otherwise set a status based on the received signals. In some embodiments, the one or more processors compare the set status to a predetermined threshold/status to determine whether the set status satisfies the privacy condition. If the set status is equal to the predetermined status (e.g., if the set status is "door open" and the predetermined status is "door open"), then the privacy condition is satisfied. In some embodiments, the privacy condition may be satisfied if a threshold is satisfied. For example, if the door is open more than 50%, then the privacy condition is satisfied. In some embodiments, the privacy condition relates to the ending of the radiotherapy treatment. For example, the PII is anonymized after the treatment is completed. The one or more processors display the icons with PII before and during the radiotherapy treatment (e.g., during alignment and administration of the therapy). However, once treatment is completed, the one or more processors receive an indication of the completed treatment from the radiotherapy machine (or other component of the radiotherapy system) and determine that a privacy condition is satisfied.

At step **530,** in response to determining that the privacy condition is satisfied, the at least one processor anonymizes at least one icon within the set of icons. As described herein, the one or more processors transmits an instruction to adjust one or more icon parameters of at least one icon of the set of icons such that the PII represented by the at least one icon is anonymized, or otherwise no longer personally identifiable. For example, the one or more processors may transmit instructions to remove the PII from display. The one or more processors may transmit instructions to relocate the PII to a location not visible to a user of the radiotherapy machine (e.g., behind a body plate). Alternatively, the one or more processors may blur the PII. Alternatively, the one or more processors may redact at least one attribute (e.g., PII represented by the icon) of the at least one icon. Redacting may include stopping the display of the attribute through one or more methods, including, but not limited to, reducing the opacity, masking with another graphic, adjusting a color of the icon's attribute, etc.

Patient identification may be a critical component of the radiotherapy workflow, ensuring that the correct treatment plan is administered to the right patient. Radiotherapy involves precise, personalized treatment protocols tailored to the patient's unique anatomy, diagnosis, and prescribed dose. Misidentification can result in administering the wrong treatment, leading to ineffective therapy and/or potential harm to the patient. Robust identification procedures, such as displaying patient-specific details (e.g., name, date of birth, and/or treatment plan) on the screen or scanning unique identifiers, help confirm that the setup aligns with the intended plan.

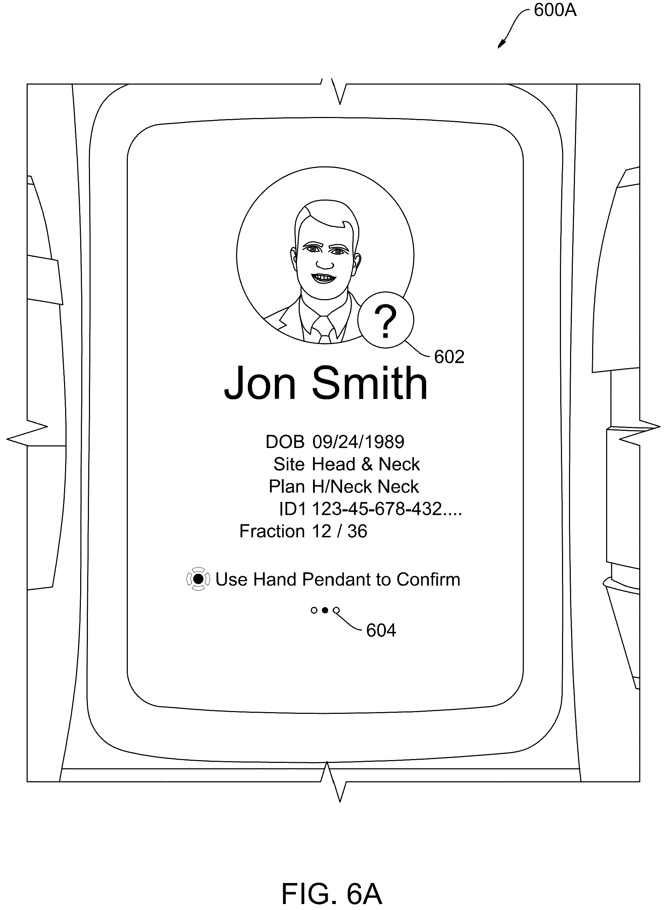

In some embodiments, a patient identification GUI may be presented before other GUIs or before any other icons are displayed. For instance, and referring to **FIG. 6A****,** a page **600A** is presented to facilitate patient identification as part of the radiotherapy workflow discussed herein.

As depicted, the page **600A** prominently displays patient-specific information to ensure accurate treatment administration. On the page **600A,** the one or more processors may display PII of the patient, such as a visual representation of the patient, such as a photograph, along with their name (e.g., "Jon Smith"). The page **600A** may also include additional patient details, such as the patient's date of birth (DOB), treatment site (e.g., "Head & Neck"), treatment plan (e.g., "H/Neck Neck"), a unique patient identifier (e.g., "ID1 123-45-678-432..."), and the current fraction of treatment being administered relative to the total prescribed sessions (e.g., "Fraction 12/36").

This structured presentation of information ensures that operators can quickly verify patient identity and treatment details, reducing the risk of misidentification or treatment errors. In some embodiments, the page **600A** may include navigation indicators, such as dots at the bottom of the screen, to signify that multiple pages of patient information can be accessed by swiping or selecting additional options.

In some embodiments, the dots **604** may signify the presence of multiple pages, each tailored to different aspects of the radiotherapy workflow. Specifically, by navigating different pages (via interacting with the dots **604),** the operator may access different types of pages where each page is directed toward a different aspect of the radiotherapy workflow. For example, "patient pages" may display patient-specific information, such as name, date of birth, treatment site, and session details, ensuring clear and accurate identification during the setup process. A non-limiting example of a patient page is the page **600A.** In contrast, other pages within the same user interface may adopt a different layout and content, focusing on machine parameters, therapist workflows, or treatment configurations. Non-limiting examples of other pages may include the pages depicted in **FIGS. 3A-B** where these pages are dedicated to displaying treatment-related information (e.g., how to set up the patient and/or the machine by adjusting the couch/gantry).

This toggle approach between different pages allows for the concept of distinct screen archetypes within a unified user interface, enabling seamless transitions between patient-oriented views and machine or therapist-oriented views. By navigating between pages, the interface can dynamically adapt its purpose and character to suit the current stage of the workflow, such as patient identification, alignment, machine parameter adjustments, and the like. In a non-limiting example, the operator may navigate back and forth between these different page types using the depicted dots **604.** For instance, after confirming the patient identity, the operator may use the pendant to interact with the depicted dots **604** and navigate to the user interface depicted in **FIG. 3A****,** where the operator can start preparing the patient and the radiotherapy machine for treatment. At any time, the operator can interact with the dots 604 in order to navigate through the different types of user interfaces (e.g., from patient-based UIs to machine-related UIs), such as to navigate back to the page **600A** (patient page) if needed.

The depicted dots **604** provide an intuitive navigation mechanism, allowing operators to switch between these archetypes as needed, ensuring that the user interface remains flexible, purpose-driven, and optimized for the specific requirements of each workflow step. This adaptability enhances operational efficiency, minimizes cognitive load, and supports a streamlined radiotherapy process.

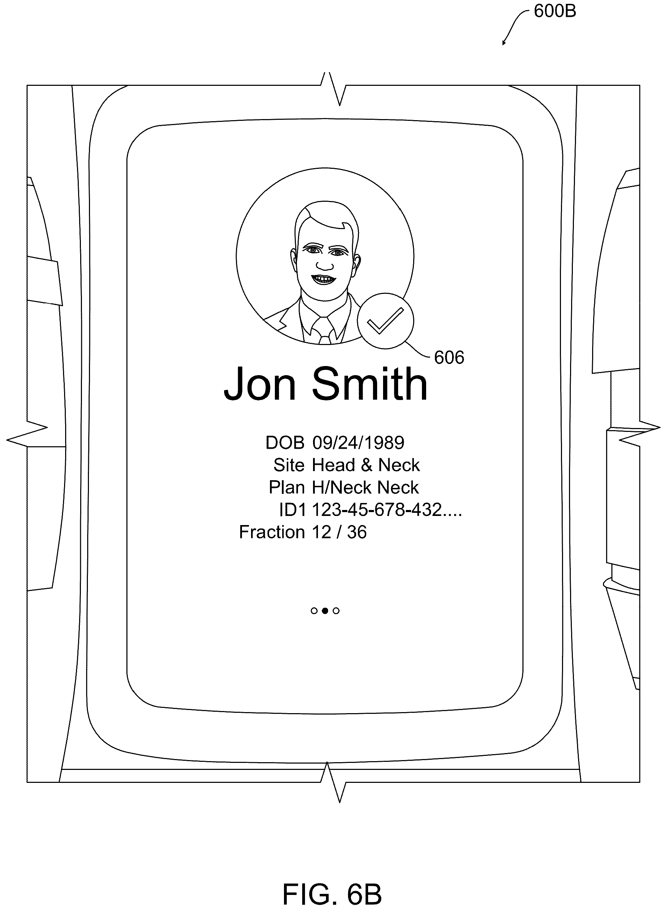

The page **600A** allows the operator to confirm the identity of the patient using various input elements discussed herein, such as by interacting with a button on the pendant. After the patient identity is confirmed, the one or more processors may display an indicator on the page **600A** using one or more methods discussed herein. In one embodiment, the indicator may be a color-coded check mark that can appear on the page **600A** in an animation style. For instance, the page **600A** includes an indicator **602,** a question mark usually in yellow to indicate that the patient identity is not confirmed. When the patient identity is confirmed, the one or more processors may transition to a page **600B** (as shown in **FIG. 6B****).** The page **600B** includes an indicator **606** which displays a check mark, usually in green, to indicate that the patient identity has been confirmed. The transition between the indicators **602** and **606** may be visually highlighted, such as using an animation style or a pulsating visual indicator, such that the change from the unconfirmed patient identity (indicator **602)** to a confirmed patient identity (indicator **606)** is easily identified by the operator.

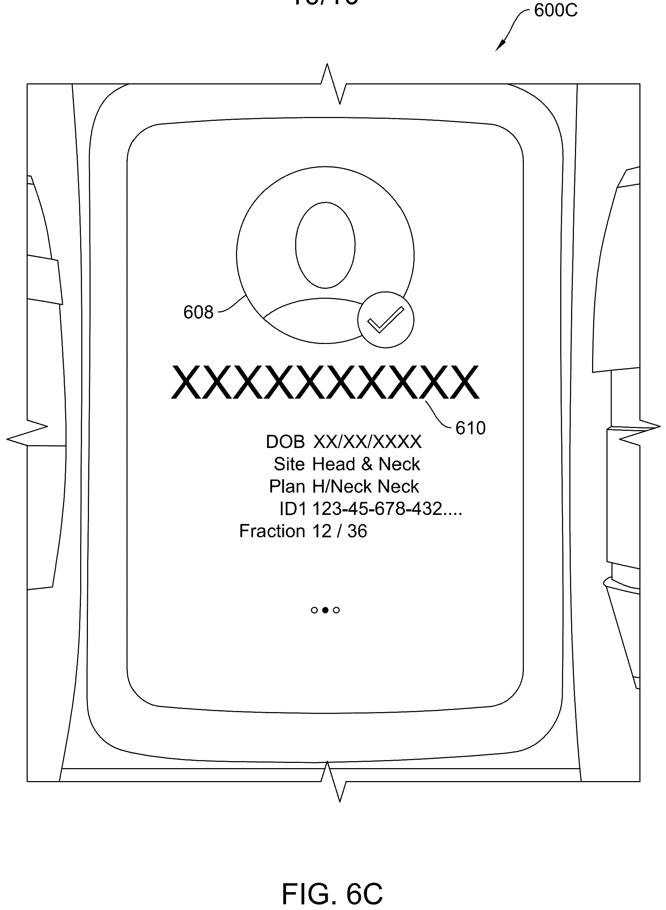

The methods and systems described herein regarding the obfuscation of PII of the patient may be implemented in page **600A** and/or the page **600B.** By way of example, the PII of the patient may be obfuscated before or after confirmation of the patient, as indicated by the indicator **602** and/or the indicator **606.** **FIG. 6C** depicts a page **600C** displaying the patient information obfuscated in accordance with the description herein, such that a generic avatar **608** replaces the visual representation of the patient and generic/anonymous text **610** replaces some or all of the other additional patient information (e.g., PII such as the patient's date of birth, name, and/or unique identifier). As described herein, the obfuscation of the patient information may be at least, or wholly, in response to one or more triggers, such as, for example, the opening of a door into the radiotherapy treatment room.

### Example clauses

Further aspects of these teachings are provided by the subject matter of the following clauses.

Clause 1. A computer-readable medium comprising instructions, that when executed by one or more processors, cause the one or more processors to: present, on a display screen located on a gantry of a radiotherapy machine, a set of icons corresponding to one or more attributes of a radiotherapy treatment implemented via the radiotherapy machine; determine that a privacy condition is satisfied; and in response to determining that the privacy condition is satisfied, anonymize at least one icon within the set of icons.

Clause 2. The computer-readable medium of clause 1, wherein the at least one icon within the set of icons comprises personally identifiable information of a patient receiving the radiotherapy treatment.

Clause 3. The computer-readable medium of clauses 1-2, wherein the privacy condition is a status change of a door of a treatment room comprising the radiotherapy machine.

Clause 4. The computer-readable medium of clauses 1-3, wherein the privacy condition is a clinician interacting with a pendant of the radiotherapy machine.

Clause 5. The computer-readable medium of clauses 1-4, wherein the privacy condition is identifying a person in a treatment room comprising the radiotherapy machine.

Clause 6. The computer-readable medium of clauses 1-5, wherein the privacy condition is an indication that the radiotherapy treatment has ended.

Clause 7. The computer-readable medium of clauses 1-6, wherein anonymizing comprises redacting at least one attribute of the at least one icon.

Clause 8. The computer-readable medium of clauses 1-7, wherein anonymizing comprises relocating the at least one icon.

Clause 9. The computer-readable medium of clauses 1-8, wherein anonymizing comprises blurring at least one attribute of the at least one icon.

Clause 10. A system comprising: one or more processors; and a computer-readable, non-transitory storage medium containing instructions that when executed by the one or more processors cause the one or more processors to perform a method comprising: presenting, on a display screen located on a gantry of a radiotherapy machine, a set of icons corresponding to one or more attributes of a radiotherapy treatment implemented via the radiotherapy machine; determining that a privacy condition is satisfied; and in response to determining that the privacy condition is satisfied, anonymizing at least one icon within the set of icons.

Clause 11. The system of clause 10, wherein the at least one icon within the set of icons comprises personally identifiable information of a patient receiving the radiotherapy treatment.

Clause 12. The system of clauses 10-11, wherein the privacy condition is a status change of a door of a treatment room comprising the radiotherapy machine.

Clause 13. The system of clauses 10-12, wherein the privacy condition is a clinician interacting with a pendant of the radiotherapy machine.

Clause 14. The system of clauses 10-13, wherein the privacy condition is identifying a person in a treatment room comprising the radiotherapy machine.

Clause 15. The system of clauses 10-14, wherein the privacy condition is an indication that the radiotherapy treatment has ended.

Clause 16. The system of clauses 10-15, wherein anonymizing comprises redacting at least one attribute of the at least one icon.

Clause 17. The system of clauses 10-16, wherein anonymizing comprises relocating the at least one icon.

Clause 18. The system of clauses 10-17, wherein anonymizing comprises blurring at least one attribute of the at least one icon.

Clause 19. A method comprising: presenting, by at least one processor on a display screen located on a gantry of a radiotherapy machine, a set of icons corresponding to one or more attributes of a radiotherapy treatment implemented via the radiotherapy machine; determining, by the at least one processor, that a privacy condition is satisfied; and in response to determining that the privacy condition is satisfied, anonymizing, by the at least one processor, at least one icon within the set of icons.

Clause 20. The method of clause 19, wherein the privacy condition is a status change of a door of a treatment room comprising the radiotherapy machine.

Clause 21: A computer program comprising instructions which, when the program is executed by a computer, cause the computer to carry out the method of any of clauses 19-20.

Clause 22: A computer-readable medium comprising a set of instructions, that when executed by one or more processors, cause the one or more processors to carry out the method of any of clauses 19-20.

The presentation of information on the display screen, the user interaction with the display screen and/or the GUI, may have the technical effect of assisting the user in performing a radiotherapy setup workflow by means of a continued and/or guided process of human-machine interaction. The systems discussed herein may be configured to assist the user in performing a radiotherapy workflow and/or a radiotherapy setup workflow, by means of a continued and/or guided process of human-machine interaction.

In this disclosure, the term "radiotherapy treatment" may refer to radiotherapy treatment and/or radiotherapy treatment setup. The method steps may refer to treatment setup, while the systems may be configured to perform treatment and/or treatment setup.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory storage medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM, or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting with the true scope and spirit being indicated by the following claims.

## Claims

1. A computer-readable medium comprising instructions, that when executed by one or more processors, cause the one or more processors to:
present, on a display screen coupled to a radiotherapy machine, a set of icons corresponding to one or more attributes of a radiotherapy treatment implemented via the radiotherapy machine;
determine that a privacy condition is satisfied; and
in response to determining that the privacy condition is satisfied, anonymize at least one icon within the set of icons.

2. A system comprising:
one or more processors; and
a computer-readable, non-transitory storage medium containing instructions that when executed by the one or more processors cause the one or more processors to perform a method comprising:
presenting, on a display screen coupled to a radiotherapy machine, a set of icons corresponding to one or more attributes of a radiotherapy treatment implemented via the radiotherapy machine;
determining that a privacy condition is satisfied; and
in response to determining that the privacy condition is satisfied, anonymizing at least one icon within the set of icons.

3. A method comprising:
presenting, by at least one processor on a display screen coupled to a radiotherapy machine, a set of icons corresponding to one or more attributes of a radiotherapy treatment implemented via the radiotherapy machine;
determining, by the at least one processor, that a privacy condition is satisfied; and
in response to determining that the privacy condition is satisfied, anonymizing, by the at least one processor, at least one icon within the set of icons.

4. The computer-readable medium of claim 1, the system of claim 2 or the method of claim 3, wherein the at least one icon within the set of icons comprises personally identifiable information of a patient receiving the radiotherapy treatment.

5. The computer-readable medium, system or method of any one of the preceding claims, wherein the privacy condition is a status change of a door of a treatment room comprising the radiotherapy machine.

6. The computer-readable medium, system or method of any one of the preceding claims , wherein the privacy condition is a clinician interacting with a pendant of the radiotherapy machine.

7. The computer-readable medium, system or method of any one of the preceding claims , wherein the privacy condition is identifying a person in a treatment room comprising the radiotherapy machine.

8. The computer-readable medium, system or method of any one of the preceding claims , wherein the privacy condition is an indication that the radiotherapy treatment has ended.

9. The computer-readable medium, system or method of any one of the preceding claims , wherein anonymizing comprises redacting at least one attribute of the at least one icon.

10. The computer-readable medium, system or method of any one of the preceding claims , wherein anonymizing comprises relocating the at least one icon.

11. The computer-readable medium, system or method of any one of the preceding claims , wherein anonymizing comprises blurring at least one attribute of the at least one icon.
